# EUROPEAN PATENT SPECIFICATION

(11) **EP 1 684 844 B1**
(45) Date of publication and mention of the grant of the patent: **26.06.2019**
(21) Application number: 04796062.0
(22) Date of filing: 22.10.2004
(51) Int. Cl.: A61M 29/00, A61M 25/06, A61M 25/00, A61B 90/00

(54) **VASCULAR PUNCTURE DEPTH LOCATOR**
GEFÄSSPUNKTIONSTIEFEN-LOKALISATOR
LOCALISATEUR DE PROFONDEUR DE PONCTION VASCULAIRE

(30) Priority: 13.11.2003 US 713530
(43) Date of publication of application: 02.08.2006
(73) Proprietor: Terumo Puerto Rico, L.L.C., Caguas (PR)
(72) Inventor: FORSBERG, Andrew, Thomas, Minneapolis, MN 55406 (US); PAPROCKI, Loran, St. Louis, MN (US)
(74) Representative: Strehl Schübel-Hopf & Partner
(86) International application number: PCT/US2004/035002
(87) International publication number: WO 2005/051475

(56) References cited:
- WO-A-02/28286
- WO-A-2004/096056
- US-A- 5 522 399
- US-A- 5 725 496
- US-A- 5 807 326
- US-A- 6 033 427
- US-B1- 6 179 863
- US-B1- 6 193 670
- US-B2- 6 547 803

## Description

### FIELD OF THE INVENTION

The present invention relates to a vascular sheath and dilator and, more particularly, to a vascular sheath and dilator having at least one puncture depth locator in the sheath.

### BACKGROUND OF THE INVENTION

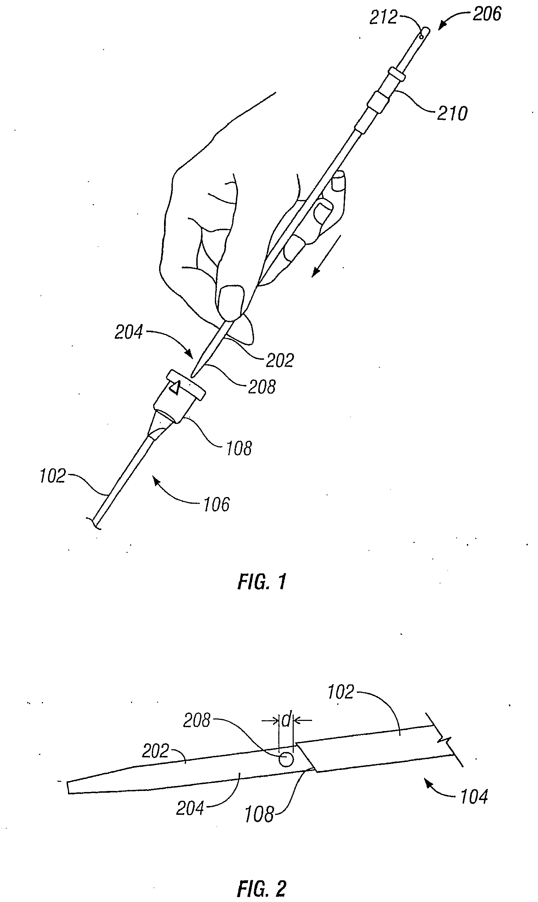

Vascular insertion of devices or arterial seals requires puncture of an arterial vessel and placement of the device or seal. FIGS. 1, 2 and 3 show a conventional insertion sheath 102 and dilator 202 useful for vascular penetration of a blood vessel 302 in a patient 304. Insertion sheath 102 has a sheath distal end 104 and a sheath proximate end 106. Sheath distal end 104 contains a tool access port, and sheath proximate end 106 has a tool junction port 108. Dilator 202 has a dilator distal end 204 and a dilator proximate end 206. Dilator distal end 204 contains an inlet port 208. Dilator proximate end 206 contains a tool junction connector 210 and a drip hole 212. Inlet port 208 is in fluid communication with drip hole 212 via a lumen (not specifically shown in FIGS. 1 and 2) contained in dilator 202.

Placing a device, such as, a vascular seal, using insertion sheath 102 and dilator 202 will now be explained. First, a puncture site 306 is located that will allow access to blood vessel 302, such as the femoral artery. Dilator 202 is placed in insertion sheath 102 so that sheath distal end 104 is substantially adjacent dilator distal end 204 and tool junction port 108 and tool junction connector 210 mate. Dilator 202- is somewhat longer than insertion sheath 102 such that inlet port 208 resides outside sheath distal end 104 by a predetermined distance.

Using conventional techniques, dilator 202 and insertion sheath 102 are inserted through puncture site 306 into blood vessel 302 of patient 304. As sheath distal end 104 penetrates blood vessel 302, blood will flow from inlet port 208 to drip hole 212 via the dilator lumen (not shown).

Blood exiting drip hole 212 indicates insertion sheath 102 has just penetrated blood vessel 302. To ensure proper placement of structure, insertion sheath 102 and dilator 202 are backed out of the vessel until blood stops flowing from drip hole 212. Next, insertion sheath 102 and dilator 202 are re-inserted in blood vessel 302 until blood starts flowing from drip hole 212. Proper depth of penetration and location of the assembly is established by continuing to insert and additional distance, for example, a doctor would insert the assembly 1 to 2 centimeters for the femoral artery. Once properly installed, the vascular seal or vascular tool can be inserted via insertion sheath 102 and introduced to blood vessel 302.

The above assembly has various drawbacks, such as, for example, over inserting the assembly. Also, because inlet port 208 is located on dilator distal end 204 it is at best only a proximate location indicator for the location of sheath distal end 104. Thus, it would be desirous to provide an improved vascular penetration depth locator. WO 02/28286 A discloses a vascular insertion assembly according to the preamble of present claim 1. Other conventional assemblies are described in WO 2004/096056 A, US 6 033 427 A, US 5 522 399 A, US 6 193 670 B1, US 5 725 496 A, US 6 179 863 B1, US 6 547 803 B2, and US 5 807 326 A.

### SUMMARY OF THE INVENTION

To attain the advantages and in accordance with the purpose of the invention, as embodied and broadly described herein, a vascular insertion assembly as defined in present claim 1 is provided. The vascular insertion assembly includes an insertion sheath and a dilator. The dilator is designed to fit snuggly in the insertion sheath. A first inlet port is located towards the sheath distal end and a first indicator is located towards the proximate end. The first indicator provides penetration indication when the first inlet port penetrates the vessel.

The foregoing and other features, utilities and advantages of the invention will be apparent from the following more particular description of a preferred embodiment of the invention as illustrated in the accompanying drawings.

### BRIEF DESCRIPTION OF THE DRAWINGS

The above and other objects and advantages of the present invention will be apparent upon consideration of the following detailed description, taken in conjunction with the accompanying drawings, in which like reference characters refer to like parts throughout, and in which:
FIG. 1 is a perspective view of a conventional insertion sheath and dilator;
FIG. 2 is a perspective view of a conventional insertion sheath and dilator;
FIG. 3 is a perspective view of a conventional insertion sheath and dilator in use;
FIG. 4 is a perspective view of an assembly consistent with the present invention;
FIG. 5 is a perspective view of a portion of an assembly consistent with the present invention;
FIG. 6 is a perspective view of a portion of an assembly consistent with the present invention;
FIG. 7 is a cross sectional view of a gauge usable with an assembly consistent with the present invention;
FIG. 8 is a front plan view of a faceplate of a gauge usable with an assembly consistent with the present invention; and
FIG. 9A to 9C shows use of an assembly consistent with the present invention.

### DETAILED DESCRIPTION

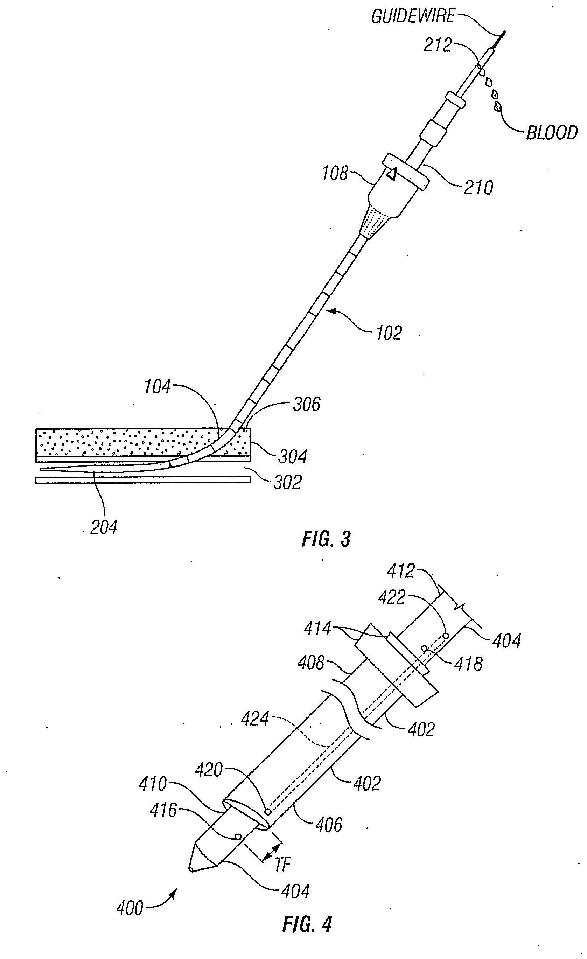

The present invention will now be described with reference to FIGS. 4-9C. Referring to FIG. 4, an assembly 400 consistent with the present invention is shown. Assembly 400 includes an insertion sheath 402 and a dilator 404. Insertion sheath 402 includes a sheath distal end 406 and a sheath proximate end 408. Dilator 404 includes a dilator distal end 410 and a dilator proximate end 412. Insertion sheath 402 and dilator 404 are coupled using a conventional mating 414. Similar to conventional assemblies (described above), dilator distal end 410 contains an inlet port 416, and dilator proximate end 412 contains a drip hole 418. Inlet port 416 and drip hole 418 are in fluid communication via a first lumen (not shown) in dilator 404 or the like. Unlike conventional assemblies, however, assembly 400 contains an over insertion indication port 420 located in sheath distal end 406, and over insertion drip hole 422 located in dilator proximate end 412 (as shown). Over insertion indication port 420 and over insertion drip hole 422 are in fluid communication via a second lumen 424 (shown in phantom) that exists separate from the first lumen. Inlet port 416 and over insertion indication port 420 can be located a predetermined distance TF to indicate assembly 400 has been inserted too far. While drip hole 418 and over insertion drip hole 422 are shown arranged sequentially for when blood would flow, alternative arrangements are possible. Also, while over insertion drip hole 422 is shown arranged on dilator proximate end 412 in the drawings, the present invention calls for an over insertion drip hole 422 arranged on sheath proximate end 408, which may actually facilitate manufacturing of assembly 400.

In use, assembly 400 would first be inserted until vessel penetration was indicated, as with the prior art device above. Using the prior art device, care must be exercised during reinsertion of the assembly approximately 1 to 2 centimeters to ensure it is not over inserted. Using assembly 400, however, over insertion indicator port 420 would provide over insertion indication when blood begins flowing from over insertion drip hole 422. Thus, assisting proper location of assembly 400 and avoiding over insertion. Distance TF could be selected to provide indication that assembly 400 is about to be over inserted (*i.e.,* blood flows from over insertion drip hole 422 prior to over insertion) or to provide indication that over insertion just occurred (*i.e.,* blood flows from over insertion drip hole 422 at or just after over insertion).

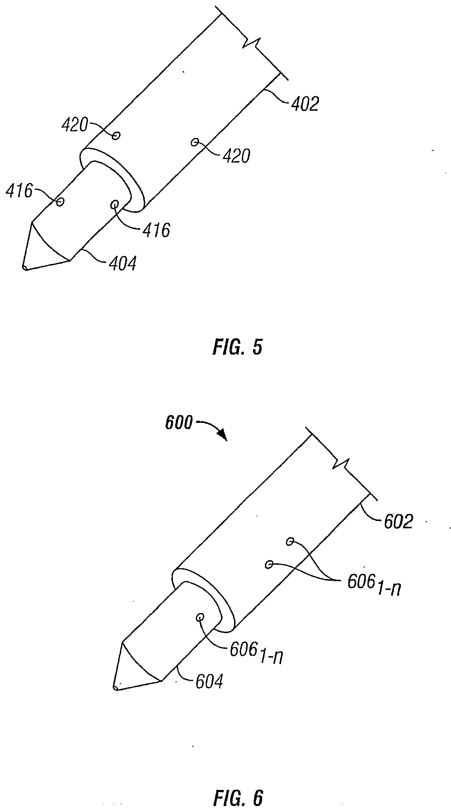

Assembly 400 is shown with inlet port 416 and drip hole 418 as the primary artery entry sensor holes. Over insertion indication port 420 and over insertion drip hole 422 are the insertion too far sensor holes. Alternative arrangements for these sensor holes are possible. For example, referring to FIG. 5, multiple inlet ports 416 could be provided in dilator 404 and multiple over insertion indication ports 420 could be provided in insertion sheath 402 to provide better indication of entry and over insertion. Moreover, as shown, the ports could be offset or staggered to accommodate an insertion angle for assembly 400.

Fluid communication is provided between ports 416 and 420 and holes 418 and 422 by separate fluid conduits. It is believed separate lumens would work well, but other types of tubes or capillaries could be used. Moreover, a single lumen having multiple and separate flow paths would work as well. Lumens could be contained in the dilator or in the sheath as a matter of design choice. Having the lumen contained in the dilator would inhibit locating when the dilator is removed.

Referring now to FIG. 6, a lower portion 600 of an insertion sheath 602 and a dilator 604 is shown. In this case, multiple ports 606₁₋ₙ are shown. One of skill in the art would now recognize that corresponding drip holes and flow paths, such as, for example, lumens, would exists, but that they are not shown for ease of reference. In this case, the ports could provide indication of initial insertion, proper insertion, and over insertion. More or less ports and drip holes could be used to provide additional indications. For example, the port on dilator 604 could be removed and location information could be provided using only the proper insertion indication and over insertion indication. Still further, the over insertion indication could be removed leaving only the proper insertion port. As can be seen, the combination of ports is limited only by the design of the insertion sheath.

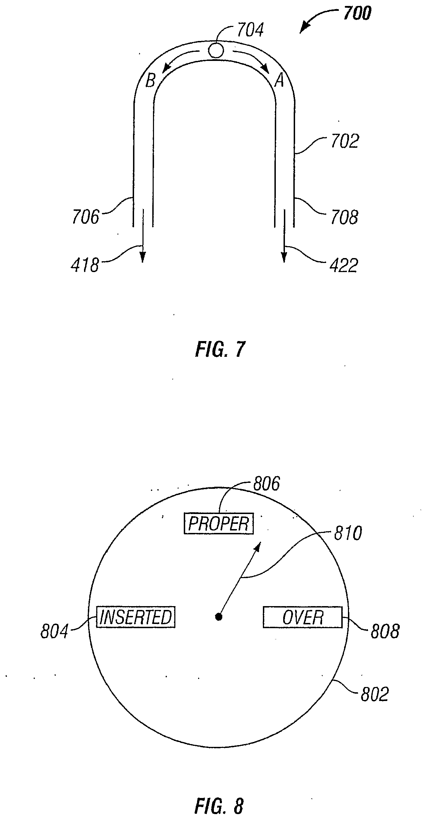

Referring to FIG. 4, location indication is provided by fluid flow out of drip hole 418 and/or over insertion drip hole 422. Alternative means of indication are possible, however. As shown by FIG. 7, a differential pressure gauge 700 could be supplied for indication. Differential pressure gauge 700, in this case, is a ball float gauge comprising a tube 702 and a suspended ball 704. Tube 702 has two access ports 706 and 708. Access port 706 could be connected to drip hole 418 and access port 708 could be connected to over insertion drip hole 422. In this case, when inlet port 416 entered a vessel, blood flow out of drip hole 418 would cause ball 704 to move towards access port 708 as shown by arrow A. If the assembly was inserted too far, blood flow out of over insertion drip hole 422 would cause ball 704 to move towards access port 706 as shown by arrow A. Thus, use of the gauge would provide visual indication without allowing blood to freely drip in the surgical arena. Alternatively, as shown by FIG. 8, differential pressure gauge 700 could have a more conventional looking faceplate 802. Faceplate 802 may provide various indications such as the point of initial insertion as shown by inserted indicator 804, proper insertion as shown by proper indicator 806, and over insertion as shown by over indicator 808. An arrow 810 or other pointer could be used to provide reference indication.

Finally, while the indicators are shown as largely mechanical in nature, electrical components could be used. For example, over insertion indication port 420 could be replaced with a fluid sensor or a pressure sensor. A wire could replace lumen 424. And a light emitting diode or the like could replace over insertion drip hole 424. Thus, when blood or some other fluid contacted the fluid sensor, an electrical signal would be sent via the wire to the LED that would light and provide over insertion indication. Alternatively, over insertion indication port 420 and lumen 424 could provide fluid communication to a fluid sensor replacing over insertion drip hole 422. In this case, when port 420 entered fluid, fluid communicated via lumen 424 would cause the fluid sensor to provide a visual indication, an audio indication, or a combination thereof.

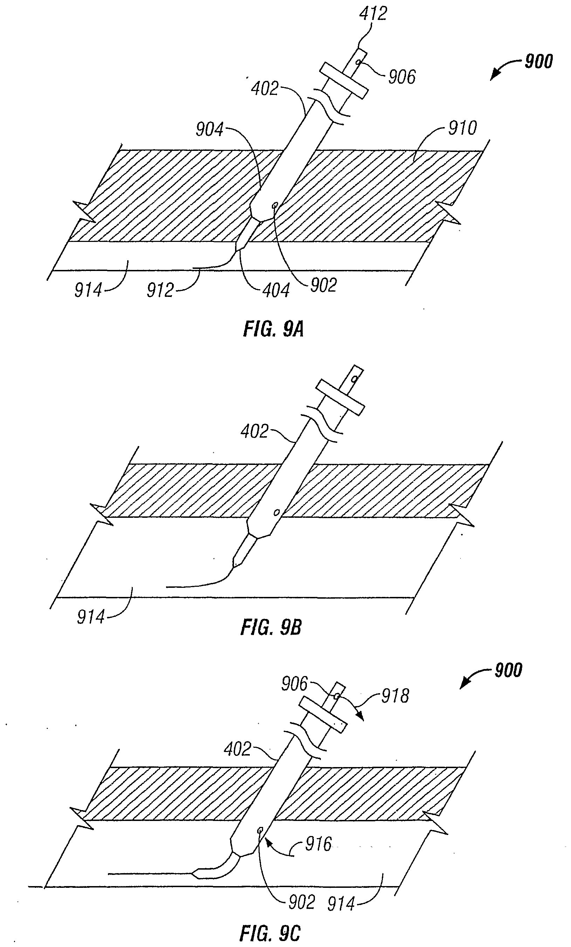

FIG. 9 shows use of an assembly 900 consistent with the present invention. In this example, assembly 900 contains dilator 404 and insertion sheath 402. Dilator 404 does not contain an inlet port and insertion sheath 402 contains proper insertion indication port 902 in a sheath distal end 904. A corresponding proper insertion drip hole 906 is located in a dilator proximate end 412. As shown, assembly 900 is inserted through a patient 910 over a guide wire 912. Dilator 404 punctures vessel 914 prior to insertion sheath 402 entering vessel 914, FIG. 9A. FIG. 9B shows insertion sheath 402 after it has entered vessel 914 but prior to achieving proper insertion depth. FIG. 9C shows insertion sheath 402 having achieved proper insertion depth. At this point, at least a portion of port 902 has entered vessel 914 and fluid flows from vessel 914 into port 902 as shown by arrow 916 and out drip hole 906 as shown by arrow 918, indicating proper insertion of insertion sheath 402. At this point, dilator 404 and guide wire 912 can be removed. If fluid communication between port 902 and drip hole 906 is provided by a lumen (not shown) in dilator 404, removal of dilator 404 removes fluid flow from drip hole 906. If the lumen (not shown) is provided in sheath 402, however, positive location information is communicated to the Doctor even after removal of dilator 404 and guide wire 912.

## Claims

1. A vascular insertion assembly (400), comprising:
an insertion sheath (402; 602);
a dilator (404; 604);
the insertion sheath (402; 602) having an inside diameter and comprising a sheath distal end (406) and a sheath proximate end (408);
the dilator (404; 604) sized to fit in the inside diameter of the insertion sheath (402; 602) comprising a dilator distal end (410) and a dilator proximate end (412), the dilator distal end (410) positionable distally beyond the sheath distal end (406);
a first inlet port (420) located in the sheath distal end (406);
a second inlet port (416) located in the dilator distal end (410);
a first indicator (422; 700) coupled with the first inlet port (420), such that when the first inlet port (420) penetrates a vessel (302) the first indicator (422; 700) provides indication of too far or proper penetration of the insertion sheath (402; 602) to the vessel (302); and
a second indicator (418; 700) located in the dilator proximate end (412) and coupled with the second inlet port (416), such that when the second inlet port (416) penetrates the vessel (302) the second indicator (418; 700) provides indication of an initial penetration of the insertion sheath (402; 602) to the vessel (302),
**characterized in that** the first indicator (422; 700) is located in the sheath proximate end (408).

2. The assembly (400) of claim 1, wherein the first inlet port (420) comprises a plurality of inlet ports (420), preferably offset to accommodate an insertion angle of the assembly (400).

3. The assembly (400) of claim 1, further comprising:
at least a third inlet port (606);
at least a third indicator (700); and
at least the third indicator (700) coupled with at least the third inlet port (606), such that when at least the third inlet port (606) penetrates the vessel (302) at least the third indicator (700) provides indication.

4. The assembly (400) of claim 3, wherein:
the second inlet port (416) and the second indicator (418; 700) provide indication of an initial penetration of the insertion sheath (402; 602) to the vessel (302);
the first inlet port (420) and the first indicator (422; 700) provide indication of proper penetration of the insertion sheath (402; 602) to the vessel (302); and
at least the third inlet port (606) and at least the third indicator (700) provide indication of too far penetration of the insertion sheath (402; 602) to the vessel (302).

5. The assembly (400) of claim 1, wherein the first indicator (422; 700) is a first drip hole (422) in fluid communication with the first inlet port (420).

6. The assembly (400) of claim 5, further comprising a first lumen (424) providing the fluid communication between the first drip hole (422) and the first inlet port (420), the first lumen (424) being preferably located in the dilator (404; 604) or in a wall of the insertion sheath (402; 602).

7. The assembly (400) of claim 1, wherein the first indicator (422; 700) is a first drip hole (422) in fluid communication with the first inlet port (420) and the second indicator (418; 700) is a second drip hole (418) in fluid communication with the second inlet port (416).

8. The assembly (400) of claim 7, further comprising:
a first lumen (424) which provides the fluid communication between the first drip hole (422) and the first inlet port (420); and
a second lumen which provides the fluid communication between the second drip hole (418) and the second inlet port (416).

9. The assembly (400) of claim 8, wherein at least one of the first lumen (424) and the second lumen is located in the dilator (404; 604) or in a wall of the insertion sheath (402; 602).

10. The assembly (400) of claim 7, further comprising:
a lumen, wherein
the lumen comprises a first flow path and a second flow path, wherein the first flow path provides the fluid communication between the first drip hole (422) and the first inlet port (420); and
a second flow path provides the fluid communication between the second drip hole (418) and the second inlet port (416).

11. The assembly (400) of claim 1, wherein the first indicator (422; 700) is a gauge (700), preferably a pressure gauge (700).

12. The assembly (400) of claim 11,
wherein the gauge (700) is a differential pressure gauge (700) and the first inlet port (420) is coupled to a first input port (708) of the differential pressure gauge (700) and the second inlet port (416) is coupled to a second input port (706) of the differential pressure gauge (700), such that the differential pressure gauge (700) indicates when the second inlet port (416) and the first inlet port (420) penetrate the vessel (302).

13. The assembly (400) of claim 12, wherein the differential pressure gauge (700) is a ball float gauge.

14. The assembly (400) of claim 13, wherein the differential pressure gauge (700) contains a faceplate (802), the faceplate (802) preferably providing penetration information.

## Patentansprüche

1. Gefäßeinführvorrichtung (400) mit
einer Einführhülse (402; 602),
einem Aufweiter (404; 604),
wobei die Einführhülse (402; 602) einen Innendurchmesser hat und ein distales Hülsenende (406) und ein proximales Hülsenende (408) aufweist,
wobei der Aufweiter (404; 604) so bemessen ist, dass er in den Innendurchmesser der Einführhülse (402; 602) passt, und ein distales Aufweiterende (410) und ein proximales Aufweiterende (412) hat, wobei das distale Aufweiterende (410) distal über das distale Hülsenende (406) hinaus positionierbar ist,
einer ersten Einlassöffnung (420), die in dem distalen Hülsenende (406) positioniert ist,
einer zweiten Einlassöffnung (416), die in dem distalen Aufweiterende (410) positioniert ist,
einem ersten Indikator (422; 700), der so mit der ersten Einlassöffnung (420) gekoppelt ist, dass bei Eindringen der ersten Einlassöffnung (420) in ein Gefäß (302) der erste Indikator (422; 700) eine Anzeige eines zu weiten oder richtigen Eindringens der Einführhülse (402; 602) in das Gefäß (302) liefert, und
einem zweiten Indikator (418; 700), der in dem proximalen Aufweiterende (412) positioniert und so mit der zweiten Einlassöffnung (416) gekoppelt ist, dass bei Eindringen der zweiten Einlassöffnung (416) in das Gefäß (302) der zweite Indikator (418; 700) eine Anzeige eines ersten Eindringens der Einführhülse (402; 602) in das Gefäß (302) liefert,
**dadurch gekennzeichnet, dass** der erste Indikator (422; 700) in dem proximalen Hülsenende (408) positioniert ist.

2. Vorrichtung (400) nach Anspruch 1, wobei die erste Einlassöffnung (420) mehrere Einlassöffnungen (420) umfasst, die vorzugsweise versetzt sind, um einen Einführwinkel der Vorrichtung (400) zu ermöglichen.

3. Vorrichtung (400) nach Anspruch 1, ferner mit
wenigstens einer dritten Einlassöffnung (606),
wenigstens einem dritten Indikator (700), und
wobei wenigstens der dritte Indikator (700) mit der wenigstens dritten Einlassöffnung (606) so gekoppelt ist, dass bei Eindringen der dritten Einlassöffnung (606) in das Gefäß (302) wenigstens der dritte Indikator (700) eine Anzeige liefert.

4. Vorrichtung (400) nach Anspruch 3, wobei
die zweite Einlassöffnung (416) und der zweite Indikator (418; 700) eine Anzeige eines ersten Eindringens der Einführhülse (402; 602) in das Gefäß (302) liefern,
die erste Einlassöffnung (420) und der erste Indikator (422; 700) eine Anzeige eines richtigen Eindringens der Einführhülse (402; 602) in das Gefäß (302) liefern, und
wenigstens die dritte Einlassöffnung (606) und wenigstens der dritte Indikator (700) eine Anzeige eines zu weiten Eindringens der Einführhülse (402; 602) in das Gefäß (302) liefern.

5. Vorrichtung (400) nach Anspruch 1, wobei der erste Indikator (422; 700) ein erstes Abtropfloch (422) in Fluidkommunikation mit der ersten Einlassöffnung (420) ist.

6. Vorrichtung (400) nach Anspruch 5, ferner mit einem ersten Lumen (424), das die Fluidkommunikation zwischen dem ersten Abtropfloch (422) und der ersten Einlassöffnung (420) bereitstellt, wobei das erste Lumen (424) vorzugsweise in dem Aufweiter (404; 604) oder in einer Wand der Einführhülse (402; 602) positioniert ist.

7. Vorrichtung (400) nach Anspruch 1, wobei der erste Indikator (422; 700) ein erstes Abtropfloch (422) in Fluidkommunikation mit der ersten Einlassöffnung (420) ist, und der zweite Indikator (418; 700) ein zweites Abtropfloch (418) in Fluidkommunikation mit der zweiten Einlassöffnung (416) ist.

8. Vorrichtung (400) nach Anspruch 7, ferner mit
einem ersten Lumen (424), das die Fluidkommunikation zwischen dem ersten Abtropfloch (422) und der ersten Einlassöffnung (420) bereitstellt, und
einem zweiten Lumen, das die Fluidkommunikation zwischen dem zweiten Abtropfloch (418) und der zweiten Einlassöffnung (416) bereitstellt.

9. Vorrichtung (400) nach Anspruch 8, wobei wenigstens eines von dem ersten Lumen (424) und dem zweiten Lumen in dem Aufweiter (404; 604) oder in einer Wand der Einführhülse (402; 602) positioniert ist.

10. Vorrichtung (400) nach Anspruch 7, ferner mit
einem Lumen, wobei
das Lumen einen ersten Strömungspfad und einen zweiten Strömungspfad aufweist, wobei der erste Strömungspfad die Fluidkommunikation zwischen dem ersten Abtropfloch (422) und der ersten Einlassöffnung (420) bereitstellt, und
der zweite Strömungspfad die Fluidkommunikation zwischen dem zweiten Abtropfloch (418) und der zweiten Einlassöffnung (416) bereitstellt.

11. Vorrichtung (400) nach Anspruch 1, wobei der erste Indikator (422; 700) ein Messinstrument (700) ist, vorzugsweise ein Druckmessinstrument (700).

12. Vorrichtung (400) nach Anspruch 11,
wobei das Messinstrument (700) ein Differentialdruck-Messinstrument (700) ist und die erste Einlassöffnung (420) mit einer ersten Einlassöffnung (708) des Differentialdruck-Messinstruments (700) verbunden ist, und die zweite Einlassöffnung (416) mit einer zweiten Einlassöffnung (706) des Differentialdruck-Messinstruments (700) verbunden ist, so dass das Differentialdruck-Messinstrument (700) anzeigt, wenn die zweite Einlassöffnung (416) und die erste Einlassöffnung (420) in das Gefäß (302) eindringen.

13. Vorrichtung (400) nach Anspruch 12, wobei das Differentialdruck-Messinstrument (700) ein Kugelschwimmer-Messinstrument ist.

14. Vorrichtung (400) nach Anspruch 13, wobei das Differentialdruck-Messinstrument (700) eine Frontscheibe (802) aufweist, und die Frontscheibe (702) vorzugsweise Eindringinformation liefert.

## Revendications

1. Ensemble (400) d'insertion vasculaire, comprenant :
une gaine d'insertion (402 ; 602) ;
un dilatateur (404 ; 604) ;
la gaine d'insertion (402 ; 602) ayant un diamètre intérieur et comprenant une extrémité distale (406) de gaine et une extrémité proximale (408) de gaine ;
le dilatateur (404 ; 604) dimensionné pour s'ajuster dans le diamètre intérieur de la gaine d'insertion (402 ; 602) comprenant une extrémité distale (410) de dilatateur et une extrémité proximale (412) de dilatateur, l'extrémité distale (410) de dilatateur positionnable distalement au-delà de l'extrémité distale (406) de gaine ;
un premier orifice d'entrée (420) situé dans l'extrémité distale (406) de gaine ;
un deuxième orifice d'entrée (416) situé dans l'extrémité distale (410) de dilatateur ;
un premier indicateur (422 ; 700) couplé avec le premier orifice d'entrée (420), de telle manière que lorsque le premier orifice d'entrée (420) pénètre un vaisseau (302), le premier indicateur (422 ; 700) fournit une indication d'une pénétration trop lointaine ou correcte de la gaine d'insertion (402 ; 602) dans le vaisseau (302) ; et
un deuxième indicateur (418 ; 700) situé dans l'extrémité proximale (412) de dilatateur et couplé avec le deuxième orifice d'entrée (416), de telle manière que lorsque le deuxième orifice d'entrée (416) pénètre le vaisseau (302), le deuxième indicateur (418; 700) fournit une indication d'une pénétration initiale de la gaine d'insertion (402 ; 602) dans le vaisseau (302),
**caractérisé en ce que** le premier indicateur (422 ; 700) est situé dans l'extrémité proximale (408) de gaine.

2. Ensemble (400) selon la revendication 1, dans lequel le premier orifice d'entrée (420) comprend une pluralité d'orifices d'entrée (420), préférablement décalés pour recevoir un angle d'insertion de l'ensemble (400).

3. Ensemble (400) selon la revendication 1, comprenant en outre :
au moins un troisième orifice d'entrée (606) ;
au moins un troisième indicateur (700) ; et
au moins le troisième indicateur (700) couplé avec au moins le troisième orifice d'entrée (606), de telle manière que lorsqu'au moins le troisième orifice d'entrée (606) pénètre le vaisseau (302), au moins le troisième indicateur (700) fournit une indication.

4. Ensemble (400) selon la revendication 3, dans lequel :
le deuxième orifice d'entrée (416) et le deuxième indicateur (418; 700) fournissent une indication d'une pénétration initiale de la gaine d'insertion (402 ; 602) dans le vaisseau (302) ;
le premier orifice d'entrée (420) et le premier indicateur (422 ; 700) fournissent une indication d'une pénétration correcte de la gaine d'insertion (402 ; 602) dans le vaisseau (302) ; et
au moins le troisième orifice d'entrée (606) et au moins le troisième indicateur (700) fournissent une indication d'une pénétration trop lointaine de la gaine d'insertion (402 ; 602) dans le vaisseau (302).

5. Ensemble (400) selon la revendication 1, dans lequel le premier indicateur (422 ; 700) est un premier trou de drainage (422) en communication de fluide avec le premier orifice d'entrée (420).

6. Ensemble (400) selon la revendication 5, comprenant en outre une première lumière (424) fournissant la communication de fluide entre le premier trou de drainage (422) et le premier orifice d'entrée (420), la première lumière (424) étant préférablement située dans le dilatateur (404 ; 604) ou dans une paroi de la gaine d'insertion (402 ; 602).

7. Ensemble (400) selon la revendication 1, dans lequel le premier indicateur (422 ; 700) est un premier trou de drainage (422) en communication de fluide avec le premier orifice d'entrée (420) et le deuxième indicateur (418 ; 700) est un deuxième trou de drainage (418) en communication de fluide avec le deuxième orifice d'entrée (416).

8. Ensemble (400) selon la revendication 7, comprenant en outre :
une première lumière (424) qui fournit la communication de fluide entre le premier trou de drainage (422) et le premier orifice d'entrée (420) ; et
une deuxième lumière qui fournit la communication de fluide entre le deuxième trou de drainage (418) et le deuxième orifice d'entrée (416).

9. Ensemble (400) selon la revendication 8, dans lequel au moins une de la première lumière (424) et de la deuxième lumière est située dans le dilatateur (404 ; 604) ou dans une paroi de la gaine d'insertion (402 ; 602).

10. Ensemble (400) selon la revendication 7, comprenant en outre :
une lumière, dans lequel
la lumière comprend un premier chemin d'écoulement et un deuxième chemin d'écoulement, dans lequel le premier chemin d'écoulement fournit la communication de fluide entre le premier trou de drainage (422) et le premier orifice d'entrée (420) ; et
un deuxième chemin d'écoulement fournit la communication de fluide entre le deuxième trou de drainage (418) et le deuxième orifice d'entrée (416).

11. Ensemble (400) selon la revendication 1, dans lequel le premier indicateur (422 ; 700) est une jauge (700), préférablement un manomètre (700).

12. Ensemble (400) selon la revendication 11,
dans lequel la jauge (700) est un manomètre différentiel (700) et le premier orifice d'entrée (420) est couplé à un premier orifice d'entrée (708) du manomètre différentiel (700) et le deuxième orifice d'entrée (416) est couplé à un deuxième orifice d'entrée (706) du manomètre différentiel (700), de telle manière que le manomètre différentiel (700) indique quand le deuxième orifice d'entrée (416) et le premier orifice d'entrée (420) pénètrent le vaisseau (302).

13. Ensemble (400) selon la revendication 12, dans lequel le manomètre différentiel (700) est une jauge à flotteur.

14. Ensemble (400) selon la revendication 13, dans lequel le manomètre différentiel (700) contient un cadran (802), le cadran (802) fournissant préférablement une information de pénétration.
